# EUROPEAN PATENT SPECIFICATION

(11) **EP 1 479 775 B1**
(45) Date of publication and mention of the grant of the patent: **07.05.2008**
(21) Application number: 03707063.8
(22) Date of filing: 25.02.2003
(51) Int. Cl.: C12N 9/10, C12P 13/08

(54) **PROCESS FOR PRODUCING L-THREONINE WITH THE USE OF BACTERIUM BELONIGING TO THE GENUS ESCHERICHIA**
VERFAHREN ZUR PRODUKTION VON L-THREONIN UNTER VERWENDUNG EINES ZUR GATTUNG ESCHERICHIA GEHÖRENDEN BAKTERIUMS
PROCEDE DE PRODUCTION DE L-THREONINE A L'AIDE D'UNE BACTERIE APPARTENANT AU GENRE ESCHERICHIA

(30) Priority: 27.02.2002 RU 2002104983
(43) Date of publication of application: 24.11.2004
(73) Proprietor: Ajinomoto Co., Inc., Tokyo 104-8315 (JP)
(72) Inventor: AKHVERDIAN, Valery, Zavenovich, 117593 Moscow (RU); SAVRASOVA, Ekaterina, Alekseevna, 107370 Moscow (RU); KAPLAN, Alla, Markovna, 107258 Moscow (RU); LOBANOV, Andrey, Olegovich, 117465 Moscow (RU); KOZLOV, Yuri, Ivanovich, 117574 Moscow (RU)
(74) Representative: Strehl Schübel-Hopf & Partner
(86) International application number: PCT/JP2003/002067
(87) International publication number: WO 2003/072786

(56) References cited:
- EP-A1- 0 116 860
- EP-A2- 0 219 027
- WO-A1-87/00202
- WO-A1-98/04715
- DE-A1- 3 823 451
- US-A- 4 980 285
- US-A- 5 175 107
- US-A- 6 004 773
- FOTHERINGHAM I G ET AL: "The cloning and sequence analysis of the aspC and tyrB genes of Escherichia coli K12" BIOCHEMICAL JOURNAL, PORTLAND PRESS, LONDON, GB, vol. 3, no. 234, 1986, pages 593-604, XP002078391 ISSN: 0264-6021
- CHASSAGNOLE C ET AL: "An integrated study of threonine-pathway enzyme kinetics in Escherichia coli" BIOCHEMICAL JOURNAL, THE BIOCHEMICAL SOCIETY, LONDON, GB, vol. 356, no. 2, 1 June 2001 (2001-06-01), pages 415-423, XP002232684 ISSN: 0264-6021
- RAIS BADR ET AL: "Threonine synthesis from aspartate in Escherichia coli cell-free extracts: Pathway dynamics" BIOCHEMICAL JOURNAL, THE BIOCHEMICAL SOCIETY, LONDON, GB, vol. 356, no. 2, 1 June 2001 (2001-06-01), pages 425-432, XP002319902 ISSN: 0264-6021
- VIOLA R E: "The central enzymes of the aspartate family of amino acid biosynthesis" ACCOUNTS OF CHEMICAL RESEARCH, ACS, WASHINGTON, DC, US, vol. 34, no. 5, May 2001 (2001-05), pages 339-349, XP002319930 ISSN: 0001-4842

## Description

### Technical Field

The present invention relates to biotechnology, specifically to a method for producing L-amino acids by fermentation and more specifically to a gene derived from bacterium *Escherichia coli*. The gene is useful for improvement of L-amino acid productivity, for example, L-threonine.

### Background Art

Conventionally L-amino acids have been industrially produced by fermentation methods utilizing strains of microorganisms obtained from natural sources or mutants of the same especially modified to enhance L-amino acid productivity.

To enhance L-amino acid productivity has been used, for example, amplification of biosynthetic genes by transformation of a microorganism by recombinant DNA (see, for example, US patent No. 4,278,765). These techniques are based on increasing the activitiy of the enzymes involved in amino acid biosynthesis and/or desensitizing the target enzymes to the feedback inhibition by the resulting L-amino acid or its by-products (see, for example, Japanese Laid-open application No56-18596 (1981), WO 95/16042 or US patent Nos. 5,661,012 and 6,040,160).

Various strains used for production of L-threonine by fermentation are known. There are strains with increased activities of the enzymes involved in L-threonine biosynthesis (US patents 5,175,107; 5,661,012; 5,705,371; 5,939,307; EP0219027), strains resistant to some chemicals such as L-threonine and its analogs (WO0114525A1, EP301572A2, US 5,376,538), strains with the target enzymes desensitized to the feedback inhibition by the resulting L-amino acid or its by-products (US patents 5,175,107; 5,661,012), strains with inactivated threonine degradation enzymes (US patents 5,939,307; 6,297,031).

The known threonine producing strain VKPM B-3996 (US patents 5,175,107, and 5,705,371) is the best threonine producer at present. For construction of the strain VKPM B-3996 several mutations and a plasmid described below were introduced in the parent strain *E. coli* K-12 (VKPM B-7). Mutant *thrA* gene (mutation thrA442) encodes aspartokinase homoserine dehydrogenase I resistant to feedback inhibition by threonine. Mutant *ilvA* gene (mutation *ilvA*442) encodes threonine deaminase with low activity leading to low rate of isoleucine biosynthesis and to a leaky phenotype of isoleucine starvation. In bacteria with *ilv*A442 mutation transcription of *thrABC* operon isn't repressed by isoleucine and therefore is very efficient for threonine production. Inactivation of *tdh* gene leads to prevention of the threonine degradation. The genetic determinant of saccharose assimilation (*scrKYABR* genes) was transferred to said strain. To increase expression of genes controlling threonine biosynthesis, plasmid pVIC40 containing mutant threonine operon *thrA442BC* was introduced in the intermediate strain TDH6. The amount of L-threonine accumulated during fermentation of the strain reaches up to 85 g/l.

The present inventors obtained, with respect to *E.coli* K-12, a mutant having a mutation, *thrR* (herein referred to as *rhtA23*) that is concerned in resistance to high concentrations of threonine or homoserine in a minimal medium (Astaurova, O.B. et al., Appl. Bioch. And Microbiol., 21, 611-616 (1985)). The mutation improved the production of L-threonine (SU Patent No. 974817), homoserine and glutamate (Astaurova, O.B. et al., Appl. Bioch. And Microbiol., 27, 556-561, 1991, EP 1013765 A) by the respective *E*. *coli* producing strain such as the strain VKPM-3996. Furthermore, the present inventors have revealed that the *rhtA* gene exists at 18 min on *E. coli* chromosome close to the *glnHPQ* operon that encodes components of the glutamine transport system, and that the *rhtA* gene is identical to ORF1 (*ybiF* gene, numbers 764 to 1651 in the GenBank accession number AAA218541, gi:440181), located between *pexB* and *ompX* genes. The unit expressing a protein encoded by the ORF1 has been designated as *rhtA* (rht: resistance to homoserine and threonine) gene. Also, the present inventors have found that the *rhtA23* mutation is an A-for-G substitution at position -1 with respect to the ATG start codon (ABSTRACTS of 17^{th} International Congress of Biochemistry and Molecular Biology in conjugation with 1997 Annual Meeting of the American Society for Biochemistry and Molecular Biology, San Francisco, California August 24-29, 1997, abstract No. 457, EP 1013765 A).

Under conditions for studying the mainstream threonine biosynthetic pathway and optimizing to a great extent, the further improvement of threonine producing strain could be done by supplementing bacterium with increased amount of distant precursors of threonine such as aspartate.

It is known that aspartate is a donor of carbon for synthesis of the amino acids of aspartate family (threonine, methionine, lysine), and diaminopimelate, a compound a constituent of the bacterial cell wall. These syntheses are performed by a complex pathway with several branch points and an extremely sensitive regulatory scheme. At the branch point of aspartate, aspartate semialdehyde, homoserine, there are as many isozymes as there are amino acids deriving from this biosynthetic step. The aspartokinase homoserine dehydrogenase I encoded by (part of *thrABC* operon) performs first and third reactions of threonine biosynthesis. Threonine and isoleucine regulate the expression of aspartokinase homoserine dehydrogenase I, and threonine inhibits both activities to catalyze the above-mentioned reactions (Escherichia coli and Salmonella, Second Edition, Editor in Chief: F.C.Neidhardt, ASM Press, Washington D.C., 1996).

Two genes are involved in the formation of aspartate - aspartate aminotransferase (aspartate transaminase) encoded by *aspC* gene and aspartase, which is a product of *aspA* gene. Aspartate aminotransferase converts oxaloacetate to aspartate. Aspartase converts fumarate to aspartate.

The effect of amplification of *aspC* gene on production of L-lysine - an amino acid of aspartate family - was disclosed. Amplification of *aspC* gene was used for L-lysine production by *E.coli* (US patent 6,040,160). Coryneform bacteria harboring an aspartokinase and enhanced DNA sequence coding for several enzymes including aspartate aminotransferase was used for L-lysine production (US patent 6,004,773).

It was noticed that aspartate aminotransferase could be useful for production of L-threonine and L-lysine by coryneform bacteria (US patent 4,980,285).

To date there is no report of using the bacterium belonging to the genus *Escherichia* with enhanced aspartate aminotransferase activity for production of L-threonine.

### Disclosure of the Invention

An object of the present invention is to enhance the productivity of L- threonine producing strains and to provide a method for producing L-threonine using these strains.

This aim was achieved by finding that the *aspC* gene encoding aspartate aminotransferase cloned on a low copy vector can enhance L-threonine production. Thus the present invention has been completed.

The present inventions are as follows:
(1) An L-threonine producing bacterium belonging to the genus *Escherichia*, wherein the bacterium has been modified to enhance an activity of aspartate aminotransferase, wherein the aspartate aminotransferase gene is originated from a bacterium belonging to the genus *Escherichia,* and encodes the following protein (A) or (B):
   (A) a protein comprising the amino acid sequence shown in SEQ ID NO: 2 in Sequence listing;
   (B) a protein comprising an amino acid sequence including deletion, substitution, insertion or addition of one or several amino acids in the amino acid sequence shown in SEQ ID NO: 2 in Sequence listing, and which has an activity of aspartate aminotransferase.
(2) The bacterium according to (1), wherein the activity of aspartate aminotransferase is enhanced by increasing the expression amount of an aspartate aminotransferase gene.
(3) The bacterium according to (1), wherein the activity of aspartate aminotransferase is increased by increasing the copy number of the aspartate aminotransferase gene or modifying an expression control sequence of the gene so that the expression of the gene is enhanced.
(4) The bacterium according to (3), wherein the copy number is increased by transformation of the bacterium with a low copy vector containing the gene.
(5) The bacterium according to (1) to (4), wherein the aspartate aminotransferase gene comprises the following DNA (a) or (b):
   (a) a DNA comprising a nucleotide sequence of the nucleotides 1 to 1191 in SEQ ID NO: 1; or
   (b) a DNA which is hybridizable with a nucleotide sequence of the nucleotides 1-1191 in SEQ ID NO:1 or a probe which can be prepared from the nucleotide sequence under stringent conditions and codes for a protein having an activity of aspartate aminotransferase.
(6) The bacterium according to (5), wherein the stringent conditions are conditions in which washing is performed at 60°C, and at a salt concentration corresponding to 1 x SSC and 0.1 % SDS.
(7) The bacterium according to (1) to (6), wherein the bacterium has been further modified to enhance expression of one or more genes selected from the group consisting of:
   the mutant *thrA* gene which codes for aspartokinase homoserine dehydrogenase I resistant to feed back inhibition by threonine;
   the *thrB* gene, which codes for homoserine kinase;
   the *thrC* gene, which codes for threonine synthase; and
   the *rhtA* gene, which codes for putative transmembrane protein.
(8) The bacterium according to (7), wherein the bacterium has been modified to increase expression amount of the mutant *thrA* gene, the *thrB* gene, the *thrC* gene and the *rhtA* gene.
(9) A method for producing L- threonine which comprises cultivating the bacterium according to (1) to (8) in a culture medium to produce and accumulate L-threonine in the culture medium, and collecting the L-threonine from the culture medium.

The present invention will be explained in detail below.

The bacterium of the present invention is an L-threonine producing bacterium belonging to the genus *Escherichia*, wherein the bacterium has been modified to enhance an activity of aspartate aminotransferase.

The bacterium belonging to the genus *Escherichia* that can be used in the present invention is not particularly limited, however for example, bacteria described by Neidhardt, F.C. et al. (*Escherichia coli* and *Salmonella typhimurium*, American Society for Microbiology, Washington D.C., 1208, Table 1) are encompassed.

In the present invention, "L-threonine producing bacterium" means a bacterium, which has an ability to accumulate L-threonine in a medium, when the bacterium of the present invention is cultured in the medium. The L-threonine producing ability may be imparted or enhanced by breeding.

The phrase "activity of aspartate aminotransferase" means activity to catalyze the reaction of formation the aspartate from oxaloacetate and L-glutamate with release of α-ketoglutarate using pyridoxal 5'-phosphate.

The phrase "modified to enhance an activity of aspartate aminotransferase" means that the activity per cell has become higher than that of a non-modified strain, for example, a wild-type strain. For example, a case where number of aspartate aminotransferase molecules per cell increases, a case where specific activity per aspartate aminotransferase molecule increases and so forth are encompassed. Furthermore, as a wild-type strain that serves as an object for comparison, for example, the *Escherichia coli* K-12 is encompassed. As a result of enhancement of intracellular activity of aspartate aminotransferase, there are obtained an effect that the amount of L-threonine accumulation in a medium increases.

Enhancement of aspartate aminotransferase activity in a bacterial cell can be attained by enhancing the expression amount of a gene coding for aspartate aminotransferase. Any of genes derived from bacteria belonging to the genus *Escherichia* and genes derived from other bacteria such as coryneform bacteria can be used as the aspartate aminotransferase gene. Among these, genes derived from bacteria belonging to the genus *Escherichia* are preferred.

As the gene coding for aspartate aminotransferase of *Escherichia coli, aspC* has already been elucidated (nucleotide numbers 983742 to 984932 in the sequence of GenBank accession NC_000913.1, gi:16128895). Therefore, *aspC* gene can be obtained by PCR (polymerase chain reaction; refer to White, T.J. *et al*., *Trends Genet*., *5*, 185 (1989)) utilizing primers prepared based on the nucleotide sequence of the gene. Genes coding for aspartate aminotransferase of other microorganisms can be obtained in a similar manner.

The *aspC* gene originated from *Escherichia coli* is exemplified by a DNA which encodes the following protein (A) or (B):
(A) a protein, which comprises the amino acid sequence shown in SEQ ID NO: 2 in Sequence listing;
(B) a protein which comprises an amino acid sequence including deletion, substitution, insertion or addition of one or several amino acids in the amino acid sequence shown in SEQ ID NO: 2 in Sequence listing, and which has an activity of aspartate aminotransferase.

The number of "several" amino acids differs depending on the position or the type of amino acid residues in the three dimensional structure of the protein. It may be 2 to 30, preferably 2 to 15, and more preferably 2 to 5 for the protein (A). This is because of the following reason. Some amino acids have high homology to one another and the difference in such an amino acid does not greatly affect the three dimensional structure of the protein and its activity. Therefore, the protein (B) may be one which has homology of not less than 30 to 50 %, preferably not less than 50 to 70 % with respect to the entire amino acid residues for constituting aspartate aminotransferase, and which has the activity of aspartate aminotransferase.

The DNA, which codes for substantially the same protein as the aspartate aminotransferase described above, may be obtained, for example, by modifying the nucleotide sequence of DNA coding for aspartate aminotransferase (SEQ ID NO: 1), for example, by means of site-directed mutagenesis so that one or more amino acid residues at a specified site involve deletion, substitution, insertion, or addition. DNA modified as described above may be obtained by the conventionally known mutation treatment. Such treatment includes treatment the DNA coding for proteins of the present invention with hydroxylamine or treatment of the bacterium harboring the DNA with UV irradiation or reagent such as N-methyl-N'-nitro-N-nitrosoguanidine or nitrous acid.

A DNA coding for substantially the same protein as aspartate aminotransferase can be obtained by expressing a DNA having such a mutation as described above in an appropriate cell, and investigating the activity of an expressed product. A DNA coding for substantially the same protein as aspartate aminotransferase can also be obtained by isolating a DNA that is hybridizable with a probe having a nucleotide sequence comprising, for example, the nucleotide sequence shown in Sequence Listing as SEQ ID NO: 1, under the stringent conditions, and codes for a protein having the aspartate aminotransferase activity, from DNA coding for aspartate aminotransferase having a mutation or from a cell harboring it. The "stringent conditions" referred to herein is a condition under which so-called specific hybrid is formed, and non-specific hybrid is not formed. It is difficult to clearly express this condition by using any numerical value. However, for example, the stringent conditions are exemplified by a condition under which DNAs having high homology, for example, DNAs having homology of not less than 50%, preferably not less than 70%, more preferably not less than 90% are hybridized with each other, but DNAs having homology lower than the above are not hybridized with each other. Alternatively, the stringent conditions are exemplified by a condition under which DNAs are hybridized with each other at a salt concentration corresponding to an ordinary condition of washing in Southern hybridization, i.e., 1 x SSC, 0.1% SDS, preferably 0.1 x SSC, 0.1% SDS, at 60°C.

A partial sequence of the nucleotide sequence of SEQ ID NO: 1 can also be used as a probe. Such a probe may be prepared by PCR using oligonucleotides produced based on the nucleotide sequence of SEQ ID NO: 1 as primers, and a DNA fragment containing the nucleotide sequence of SEQ ID NO: 1 as a template. When a DNA fragment in a length of about 300 bp is used as the probe, the conditions of washing for the hybridization consist of, for example, 50°C, 2 x SSC and 0.1% SDS.

The substitution, deletion, insertion, or addition of nucleotide as described above also includes mutation, which naturally occurs (mutant or variant), for example, on the basis of the individual difference or the difference in species or genus of bacterium, which harbors aspartate aminotransferase.

Transformation of a bacterium with DNA coding for protein means introduction of the DNA into bacterium cell for example by conventional methods to increase expression of the gene coding for the protein of the present invention and to enhance the activity of the protein in the bacterial cell.

Methods of enhancing gene expression include increasing the gene copy number. Introduction of a gene into a vector that is able to function in a bacterium belonging to the genus *Escherichia* increases copy number of the gene. For such purposes low copy vectors can be preferably used. The low-copy vector is exemplified by pSC101, pMW118, pMW119 and the like.

Enhancing gene expression can also be achieved by introduction of multiple copies of the gene into bacterial chromosome by, for example, method of homologous recombination, or the like. As the method of transformation, any known method that has hitherto been reported can be employed. For instance, a method of treating recipient cells with calcium chloride so as to increase the permeability of DNA, which has been reported for *Escherichia coli* K-12 (Mandel, M. and Higa, A., J. Mol. Biol., 53, 159 (1970)), may be used.

On the other hand, enhancing gene expression can also be achieved by placing the DNA of the present invention under the control of a potent promoter. For example, *lac* promoter, *trp* promoter, *trc* promoter, P_{R}, P_{L} promoters of lambda phage are known as potent promoters. Using the potent promoter can be combined with the multiplication of gene copies.

Alternatively, a promoter can be enhanced by, for example, introducing a mutation into the promoter to increase a transcription level of a gene located downstream of the promoter. Furthermore, it is known that substitution of several nucleotides in the spacer between ribosome binding site (RBS) and start codon and especially the sequences immediately upstream of the start codon profoundly affect the mRNA translatability. For example, a 20-fold range in the expression levels was found, depending on the nature of the three nucleotides preceding the start codon (Gold et al., Annu. Rev. Microbiol., 35, 365-403, 1981; Hui et al., EMBO J., 3, 623-629, 1984). Earlier, the inventors of the present invention showed the *rhtA23* mutation is an A-for-G substitution at position -1 with respect to the ATG start codon (ABSTRACTS of 17^{th} International Congress of Biochemistry and Molecular Biology in conjugation with 1997 Annual Meeting of the American Society for Biochemistry and Molecular Biology, San Francisco, California August 24-29, 1997, abstract No. 457). Therefore, it may be suggested that *rhtA23* mutation enhances the *rhtA* gene expression and, as a consequence, increases the level of resistance to threonine, homoserine and some other substances transported out of cells.

Moreover, it is also possible to introduce nucleotide substitution into a promoter region of the aspartate aminotransferase gene on the bacterial chromosome so that it should be modified into a stronger one. Alteration of the expression control sequence can be performed, for example, in the same manner as the gene substitution using a temperature sensitive plasmid, as disclosed in International Patent Publication WO00/18935 and Japanese Patent Publication No. 1-215280.

Increasing copy number of aspartate aminotransferase gene can also be achieved by introducing multiple copies of the aspartate aminotransferase gene into chromosomal DNA of bacterium. In order to introduce multiple copies of the aspartate aminotransferase gene into the bacterial chromosome, homologous recombination is carried out by using a sequence whose multiple copies exist in the chromosomal DNA as targets. As sequences whose multiple copies exist in the chromosomal DNA, repetitive DNA, inverted repeats existing at the end of a transposable element can be used. Also, as disclosed in Japanese Patent Laid-open No. 2-109985, it is possible to incorporate the aspartate aminotransferase gene into transposon, and allow it to be transferred to introduce multiple copies of the gene into the chromosomal DNA.

Methods for preparation of plasmid DNA, digestion and ligation of DNA, transformation, selection of an oligonucleotide as a primer and the like may be ordinary methods well known to one skilled in the art. These methods are described, for instance, in Sambrook, J., Fritsch, E.F., and Maniatis, T., "Molecular Cloning A Laboratory Manual, Second Edition", Cold Spring Harbor Laboratory Press (1989).

The bacterium of the present invention can be obtained by introduction of the aforementioned DNAs into bacterium inherently having the ability to produce L-threonine. Alternatively, the bacterium of the present invention can be obtained by imparting the ability to produce L- threonine to the bacterium already harboring the DNAs.

As a parent strain which is to be enhanced in activity of the aspartate aminotransferase encoded by *aspC* gene, the threonine producing bacteria belonging to the genus *Escherichia* such as *E. coli* strain VKPM B-3996 (US Patent 5, 175, 107, US patent 5,705,371), *E. coli* strain NRRL-21593 (US Patent 5,939,307), *E*. *coli* strain FERM BP-3756 (US patent 5,474,918), *E. coli* strains FERM BP-3519 and FERM BP-3520 (US patent 5,376,538), *E. coli* strain MG442 (Gusyatiner et al., Genetika (in Russian), 14, 947-956 (1978)), *E*. *coli* strains VL643 and VL2055 (EP 1149911 A)and the like may be used.

The strain B-3996 is deficient in *thrC* gene and is sucrose-assimilative, in which *ilvA* gene has a leaky mutation. The strain has a mutation in *rhtA* gene, which confers resistance to high concentration of threonine or homoserine. The strain B-3996 harbors the plasmid pVIC40 which had been obtained by inserting *thrA*BC* operon including mutant *thrA* gene encoding aspartokinase homoserine dehydrogenase I which is substantially desensitized feedback inhibition by threonine into RSF1010-derived vector. The strain B-3996 was deposited on April 7, 1987 in Russian National Collection of Industrial Microorganisms (VKPM) (Dorozhny proezd. 1, Moscow 113545, Russian Federation) under the accession number B-3996.

The bacterium of the present invention is preferably further modified to enhance expression of one or more of the following genes as well as *aspC* gene:
the mutant *thrA* gene which codes for aspartokinase homoserine dehydrogenase I resistant to feed back inhibition by threonine;
   - the *thrB* gene which codes for homoserine kinase;
   - the *thrC* gene which codes for threonine synthase;
   - Another preferred embodiment of the bacterium is modified to enhance the *rhtA* gene, which codes for putative transmembrane protein in addition to enhancement of *aspC* gene. The most preferred embodiment of the bacterium is modified to increase expression amount of the *aspC* gene, the mutant *thrA* gene, the *thrB* gene, the *thrC* gene and the *rhtA* gene.

The method for producing L-threonine of the present invention comprises the steps of cultivating the bacterium of the present invention in a culture medium, to allow L-threonine to be produced and accumulated in the culture medium, and collecting L-threonine from the culture medium.

In the present invention, the cultivation, the collection and purification of L-amino acid from the medium and the like may be performed in a manner similar to the conventional fermentation method wherein an amino acid is produced using a microorganism.

The medium used for culture may be either a synthetic medium or a natural medium, so long as the medium includes a carbon source and a nitrogen source and minerals and, if necessary, appropriate amounts of nutrients which the microorganism requires for growth. The carbon source may include various carbohydrates such as glucose and sucrose, and various organic acids. Depending on the mode of assimilation of the used microorganism, alcohol including ethanol and glycerol may be used. As the nitrogen source, various ammonium salts such as ammonia and ammonium sulfate, other nitrogen compounds such as amines, a natural nitrogen source such as peptone, soybean-hydrolysate, and digested fermentative microorganism are used. As minerals, potassium monophosphate, magnesium sulfate, sodium chloride, ferrous sulfate, manganese sulfate, calcium chloride, and the like are used. As vitamins, thiamine, yeast extract and the like are used.

The cultivation is performed preferably under aerobic conditions such as a shaking culture, and stirring culture with aeration, at a temperature of 20 to 40 °C, preferably 30 to 38 °C. The pH of the culture is usually between 5 and 9, preferably between 6.5 and 7.2. The pH of the culture can be adjusted with ammonia, calcium carbonate, various acids, various bases, and buffers. Typically, a 1 to 5-day cultivation leads to the accumulation of the target L-amino acid in the liquid medium.

After cultivation, solids such as cells can be removed from the liquid medium by centrifugation or membrane filtration, and then L- threonine can be collected and purified by ion-exchange, concentration and crystallization methods.

### Best Mode for Carrying Out the Invention

The present invention will be more concretely explained below with reference to Examples.

### Example 1: Cloning of aspC gene from E. coli into pMW119 vector

The *aspC* gene was obtained from chromosomal DNA of the *E. coli* strain K-12 by PCR using primers shown in SEQ ID NOs: 3 and 4 in the Sequence listing. The obtained DNA fragment was treated with resrtiction enzymes *Pvu*II and *EcoRI* and ligated to the stable low copy plasmid pMW119 (replicon pSC101) previously treated with resrtiction enzymes *Hinc*II and *Eco*RI under control of P_{lac} promoter. Thus, the pMW-P_{lac}-aspC plasmid was obtained.

Also, *aspC* gene was placed under control of the strong P_{R} promoter of the phage lambda instead of P_{lac} promoter. A DNA duplex containing P_{R} promoter was formed using chemically synthesized 5'-phosphorylated oligonucleotides shown in the SEQ ID Nos: 5 and 6 in the Sequence listing. Then, the DNA duplex was ligated to the pMW-P_{lac}-aspC plasmid previously treated with resrtiction enzymes *Pvu*II and *Hind*III*.* Thus the plasmid pM-P_{R}-aspC was constructed.

Non-regulated high level of *aspC* gene expression could be achieved using these plasmids. The plasmids pMW-P_{lac}-aspC and pM-P_{R}-aspC are compatible with plasmid pVIC40 (replicon RSF1010), therefore two plasmids pVIC40 and pMW-P_{lac}-aspC or pVIC40 and pM-P_{R}-aspC could be maintained in the bacteria simultaneously.

Each of the pMW-P_{lac}-aspC and pM-P_{R}-aspC plasmid was introduced into streptomycin-resistant threonine producer *E*. *coli* strain B-3996 (US patent 5,175,107). Thus the strains B-3996(pMW-P_{lac}-aspC) and B-3996(pM-P_{R}-aspC) were obtained.

### Example 2 Effect of the aspC gene amplification on threonine production

The *E.coli* strains B-3996(pMW-P_{lac}-aspC) and B-3996(pM-P_{R}-aspC) were grown for 18-24 hours at 37 °C on L-agar plates containing streptomycin (100 µg/ml). Then one loop of the cells was transferred to 50 ml of L-broth of the following composition: trypton - 10g/l, yeast extract - 5 g/l, NaCl - 5 g/l. The cells (50 ml, OD₅₄₀ - 2 o.u.) grown at 37 °C within 5 hours on shaker (240 rpm) was used for seeding 450 ml of the medium for fermentation. The batch fermentation was performed in laboratory fermenters having a capacity of 1.0 1 under aeration (1/1 vvm) with stirring at a speed of 1200 rpm at 37 °C. The pH value was maintained automatically at 6.6 using 8% ammonia liquor. The results are presented in Table 1.

The composition of the fermentation medium (g/l):

| | |
|---|---|
| Sucrose | 100.0 |
| NH₄Cl | 1.75 |
| KH₂PO₄ | 1.0 |
| MgSO₄ · 7H₂O | 0.8 |
| FeSO₄ · 7H₂O | 0.01 |
| MnSO₄ · 5H₂O | 0.01 |
| Mameno(TN) | 0.15 |
| Betaine | 1.0 |
| L-isoleucine | 0.2 |

Sucrose and magnesium sulfate are sterilized separately. pH is adjusted to 6.6.

**Table 1**

| Strain | Additional *aspC* gene | Time, hours | OD₅₄₀ | Threonine, g/l |
|---|---|---|---|---|
| | | 19.1 | 34.6 | 45.0 |
| B-3996 | - | | | |
| | | 18.4 | 33.8 | 43.8 |
| B-3996 (pMW-P_{lac}-aspC) | + | 17.5 | 29.8 | 45.2 |
| | | 18.5 | 30.6 | 45.8 |
| | | | | |
| | | 18.5 | 30.8 | 45.9 |
| | | 18.3 | 30.0 | 46.2 |
| | | 18.2±0.5 | 30.3±0.5 | 45.9±0.4 |
| B-3996 (pM-P_{R}-aspC) | + | 16.0 | 32.0 | 45.4 |
| | | 18.0 | 30.8 | 46.5 |
| | | 17.8 | 29.4 | 45.8 |
| | | 18.3 | 30.0 | 45.2 |
| | | 17.9±1.0 | 30.4±1.1 | 45.6±0.6 |

### Industrial Applicability

According to the present invention, L-threonine can be efficiently produced.

## Claims

1. An L-threonine producing bacterium belonging to the genus *Escherichia*, wherein the bacterium has been modified to enhance the activity of aspartate aminotransferase, wherein said aspartate aminotransferase gene is originated from a bacterium belonging to the genus *Escherichia* and encodes the following protein (A) or (B):
(A) a protein comprising the amino acid sequence shown in SEQ ID NO: 2 in Sequence listing;
(B) a protein comprising an amino acid sequence including deletion, substitution, insertion or addition of one or several amino acids in the amino acid sequence shown in SEQ ID NO: 2 in Sequence listing, and which has an activity of aspartate aminotransferase.

2. The bacterium of claim 1, wherein said activity of aspartate aminotransferase is enhanced by increasing the expression amount of the aspartate aminotransferase gene.

3. The bacterium of claim 1, wherein said activity of aspartate aminotransferase is increased by increasing the copy number of the aspartate aminotransferase gene, or modifying an expression control sequence of the gene so that said expression of said gene is enhanced.

4. The bacterium of claim 3, wherein the copy number is increased by transformation of said bacterium with a low copy vector containing said gene.

5. The bacterium of any one of claims 1 to 4, wherein the aspartate aminotransferase gene comprises the following DNA (a) or (b):
(a) a DNA comprising a nucleotide sequence of the nucleotides 1 to 1191 in SEQ ID NO: 1; or
(b) a DNA which is hybridizable with a nucleotide sequence of the nucleotides 1-1191 in SEQ ID NO:1 or a probe which can be prepared from said nucleotide sequence under stringent conditions and codes for a protein having an activity of aspartate aminotransferase.

6. The bacterium of claim 5, wherein said stringent conditions are conditions in which washing is performed at 60°C, and at a salt concentration corresponding to 1 x SSC and 0.1 % SDS.

7. The bacterium of any of claims 1 to 6, wherein said bacterium has been further modified to enhance expression of one or more genes selected from the group consisting of:
the mutant *thr*A gene which codes for aspartokinase homoserine dehydrogenase I resistant to feed back inhibition by threonine;
the *thrB* gene, which codes for homoserine kinase;
the *thrC* gene, which codes for threonine synthase; and
the *rht*A gene, which codes for putative transmembrane protein.

8. The bacterium of claim 7, wherein said bacterium has been modified to increase expression amount of said mutant *thrA* gene, the *thrB* gene, the *thrC* gene and the *rhtA* gene.

9. A method for producing L- threonine which comprises cultivating the bacterium of any of claims 1 to 8 in a culture medium to produce and accumulate L-threonine in the culture medium, and collecting the L-threonine from the culture medium.

## Patentansprüche

1. L-Threonin produzierendes Bakterium der Gattung Escherichia, wobei das Bakterium modifiziert worden ist, um die Aktivität von Aspartataminotransferase zu verstärken, wobei die Aspartataminotransferase aus einem Bakterium der Gattung Escherichia stammt und für das folgende Protein (A) oder (B) kodiert:
(A) ein Protein, das die in Sequenz ID NO:2 des Sequenzprotokolls gezeigte Aminosäuresequenz enthält;
(B) ein Protein, das eine Aminosäuresequenz, einschließlich Deletion, Substitution, Insertion oder Addition von einer oder mehreren Aminosäuren in der in Sequenz ID NO:2 des Sequenzprotokolls gezeigten Aminosäuresequenz enthält und die Aktivität von Aspartataminotransferase hat.

2. Bakterium nach Anspruch 1, wobei die Aktivität von Aspartataminotransferase durch Erhöhen der exprimierten Menge des Aspartataminotransferasegens verstärkt ist.

3. Bakterium nach Anspruch 1, wobei die Aktivität von Aspartataminotransferase durch Erhöhen der Kopienzahl des Aspartataminotransferasegens oder durch Modifizieren einer Expressionskontrollsequenz des Gens erhöht ist, so dass die Expression des Gens verstärkt wird.

4. Bakterium nach Anspruch 3, wobei die Kopienzahl durch Transformation des Bakteriums mit einem das Gen enthaltenden Vektor mit niedriger Kopienzahl erhöht ist.

5. Bakterium nach einem der Ansprüche 1 bis 4, wobei das Aspartataminotransferasegen die folgende DNA (A) oder (B) enthält:
(a) eine DNA, die eine Nucleotidsequenz der Nucleotide 1 bis 1191 in Sequenz ID NO:1 enthält; oder
(b) eine DNA, die mit einer Nucleotidsequenz der Nucleotide 1 bis 1191 der Sequenz ID NO:1 oder einer Sonde hybridisierbar ist, die aus der Nucleotidsequenz unter stringenten Bedingungen hergestellt werden kann und für ein Protein mit einer Aktivität von Aspartataminotransferase kodiert.

6. Bakterium nach Anspruch 5, wobei die stringenten Bedingungen Bedingungen sind, bei denen das Waschen bei 60°C und bei einer 1 x SSC entsprechenden Salzkonzentration sowie 0,1 % SDS durchgeführt wird.

7. Bakterium nach einem der Ansprüche 1 bis 6, wobei das Bakterium außerdem modifiziert worden ist, um die Expression eines oder mehrerer Gene zu verstärken, die aus der folgenden Gruppe ausgewählt sind:
dem mutierten thrA-Gen, das für Aspartokinase Homoserindehydrogenase 1 kodiert, die gegenüber Rückkopplungshemmung durch Threonin beständig ist;
dem thrB-Gen, das für Homoserinkinase kodiert;
dem thrC-Gen, das für Threoninsynthase kodiert; und
dem rhtA-Gen, das für ein mutmaßliches Transmembranprotein kodiert.

8. Bakterium nach Anspruch 7, wobei das Bakterium modifiziert worden ist, um die exprimierte Menge des mutierten thrA-Gens, des thrB-Gens, des thrC-Gens und des rhtA-Gens zu erhöhen.

9. Verfahren zur Produktion von L-Threonin, welches das Kultivieren des Bakteriums nach einem der Ansprüche 1 bis 8 in einem Kulturmedium unter Produktion und Anhäufung von L-Threonin in dem Kulturmedium und das Gewinnen des L-Threonins aus dem Kulturmedium umfasst.

## Revendications

1. Bactérie produisant de la L-thréonine et appartenant au genre Escherichia, dans laquelle la bactérie a été modifiée pour renforcer l'activité de l'aspartate aminotransférase, ledit gène de l'aspartate aminotransférase provenant d'une bactérie appartenant au genre Escherichia et codant pour la protéine (A) ou (B) suivante :
(A) une protéine comprenant la séquence d'acides aminés représentée par la SEQ ID n° 2 dans la liste des séquences ;
(B) une protéine composée d'une séquence d'acides aminés comprenant la délétion, la substitution, l'insertion ou l'addition d'un ou de plusieurs acides aminés dans la séquence d'acides aminés représentée par la SEQ ID n° 2 dans la liste des séquences, et qui présente une activité d'aspartate aminotransférase.

2. Bactérie selon la revendication 1, dans laquelle ladite activité de l'aspartate aminotransférase est renforcée par le biais d'une augmentation de la quantité d'expression du gène de l'aspartate aminotransférase.

3. Bactérie selon la revendication 1, dans laquelle ladite activité de l'aspartate aminotransférase est accrue par le biais de l'augmentation du nombre de copies du gène de l'aspartate aminotransférase, ou par le biais de la modification d'une séquence de régulation de l'expression du gène qui permette de renforcer ladite expression dudit gène.

4. Bactérie selon la revendication 3, dans laquelle le nombre de copies est accru par le biais de la transformation de ladite bactérie avec un vecteur à faible nombre de copies contenant ledit gène.

5. Bactérie selon l'une quelconque des revendications 1 à 4, dans laquelle le gène de l'aspartate aminotransférase comprend l'ADN (a) ou (b) suivant :
(a) un ADN comprenant un séquence nucléotidique constituée des nucléotides 1 à 1191 représentés par la SEQ ID N° 1 ; ou
(b) un ADN que l'on peut hybrider avec une séquence nucléotidique constituée des nucléotides 1 à 1191 représentés par la SEQ ID N° 1 ou avec une sonde que l'on peut préparer à partir de ladite séquence nucléotidique dans des conditions stringentes, et qui code pour une protéine ayant une activité d'aspartate aminotransférase.

6. Bactérie selon la revendication 5, dans laquelle lesdites conditions stringentes sont des conditions dans lesquelles on effectue le lavage à 60 °C et avec une concentration de sels correspondant à du SSC 1x et à 0,1 % de SDS.

7. Bactérie selon l'une quelconque des revendications 1 à 6, dans laquelle ladite bactérie a encore été modifiée dans le but de renforcer l'expression d'un ou de plusieurs gènes choisis dans le groupe constitué des gènes suivants :
- le gène thrA mutant, qui code pour une aspartatokinase-homosérine déshydrogénase I résistant à une inhibition par rétroaction de la thréonine ;
- le gène thrB, qui code pour l'homosérine kinase ;
- le gène thrC, qui code pour la thréonine synthase ; et
- le gène rhtA, qui code pour une protéine transmembranaire putative.

8. Bactérie selon la revendication 7, dans laquelle ladite bactérie a été modifiée pour augmenter la quantité d'expression desdits gène thrA mutant, gène thrB, gène thrC et gène rhtA.

9. Procédé pour la production de L-thréonine, qui comprend la culture de la bactérie selon l'une quelconque des revendications 1 à 8 dans un milieu de culture permettant de produire et d'accumuler la L-thréonine dans le milieu de culture, et la récupération de la L-thréonine à partir du milieu de culture.


### SEQUENCE LISTING

<110> Ajinomoto Co., Inc.
<120> METHOD FOR PRODUCING L-THREONINE USING BACTERIA BELONGING TO THE GENUS ESCHERICHIA
<130> C042AYOP1313
<140>
   <141> 2003-02-25
<150> RU 2002104983
   <151> 2002-02-27
<160> 6
<170> Patent In Ver. 2. 1
<210> 1
   <211> 1191
   <212> DNA
   <213> Escherichia coli
<220>
   <221> CDS
   <222> (1) .. (1191)
<400> 1
   atg ttt gag aac att acc gcc gct cct gcc gac ccg att ctg ggc ctg     48
   Met Phe Glu Asn Ile Thr Ala Ala Pro Ala Asp Pro Ile Leu Gly Leu
     1               5                  10                  15
   gcc gat ctg ttt cgt gcc gat gaa cgt ccc ggc aaa att aac ctc ggg     96
   Ala Asp Leu Phe Arg Ala Asp Glu Arg Pro Gly Lys Ile Asn Leu Gly
                20                  25                  30
   att ggt gtc tat aaa gat gag acg ggc aaa acc ccg gta ctg acc agc    144
   Ile Gly Val Tyr Lys Asp Glu Thr Gly Lys Thr Pro Val Leu Thr Ser
            35                  40                  45
   gtg aaa aag gct gaa cag tat ctg ctc gaa aat gaa acc acc aaa aat    192
   Val Lys Lys Ala Glu Gln Tyr Leu Leu Glu Asn Glu Thr Thr Lys Asn
        50                  55                  60
   tac ctc ggc att gac ggc atc cct gaa ttt ggt cgc tgc act cag gaa    240
   Tyr Leu Gly Ile Asp Gly Ile Pro Glu Phe Gly Arg Cys Thr Gln Glu
   65                  70                  75                  80
   ctg ctg ttt ggt aaa ggt agc gcc ctg atc aat gac aaa cgt gct cgc     288
   Leu Leu Phe Gly Lys Gly Ser Ala Leu Ile Asn Asp Lys Arg Ala Arg
                   85                  90                  95
   acg gca cag act ccg ggg ggc act ggc gca cta cgc gtg gct gcc gat     336
   Thr Ala Gln Thr Pro Gly Gly Thr Gly Ala Leu Arg Val Ala Ala Asp
               100                 105                 110
   ttc ctg gca aaa aat acc agc gtt aag cgt gtg tgg gtg agc aac cca     384
   Phe Leu Ala Lys Asn Thr Ser Val Lys Arg Val Trp Val Ser Asn Pro
           115                 120                 125
   agc tgg ccg aac cat aag agc gtc ttt aac tct gca ggt ctg gaa gtt     432
   Ser Trp Pro Asn His Lys Ser Val Phe Asn Ser Ala Gly Leu Glu Val
       130                 135                 140
   cgt gaa tac gct tat tat gat gcg gaa aat cac act ctt gac ttc gat     480
   Arg Glu Tyr Ala Tyr Tyr Asp Ala Glu Asn His Thr Leu Asp Phe Asp
   145                 150                 155                 160
   gca ctg att aac agc ctg aat gaa gct cag gct ggc gac gta gtg ctg     528
   Ala Leu Ile Asn Ser Leu Asn Glu Ala Gln Ala Gly Asp Val Val Leu
                   165                 170                 175
   ttc cat ggc tgc tgc cat aac cca acc ggt atc gac cct acg ctg gaa     576
   Phe His Gly Cys Cys His Asn Pro Thr Gly Ile Asp Pro Thr Leu Glu
               180                 185                 190
   caa tgg caa aca ctg gca caa ctc tcc gtt gag aaa ggc tgg tta ccg     624
   Gln Trp Gln Thr Leu Ala Gln Leu Ser Val Glu Lys Gly Trp Leu Pro
           195                 200                 205
   ctg ttt gac ttc gct tac cag ggt ttt gcc cgt ggt ctg gaa gaa gat     672
   Leu Phe Asp Phe Ala Tyr Gln Gly Phe Ala Arg Gly Leu Glu Glu Asp
       210                 215                 220
   gct gaa gga ctg cgc gct ttc gcg gct atg cat aaa gag ctg att gtt     720
   Ala Glu Gly Leu Arg Ala Phe Ala Ala Met His Lys Glu Leu Ile Val
   225                 230                 235                 240
   gcc agt tcc tac tct aaa aac ttt ggc ctg tac aac gag cgt gtt ggc     768
   Ala Ser Ser Tyr Ser Lys Asn Phe Gly Leu Tyr Asn Glu Arg Val Gly
                   245                 250                 255
   gct tgt act ctg gtt gct gcc gac agt gaa acc gtt gat cgc gca ttc     816
   Ala Cys Thr Leu Val Ala Ala Asp Ser Glu Thr Val Asp Arg Ala Phe
               260                 265                 270
   agc caa atg aaa gcg gcg att cgc gct aac tac tct aac cca cca gca     864
   Ser Gln Met Lys Ala Ala Ile Arg Ala Asn Tyr Ser Asn Pro Pro Ala
           275                 280                 285
   cac ggc gct tct gtt gtt gcc acc atc ctg agc aac gat gcg tta cgt     912
   His Gly Ala Ser Val Val Ala Thr Ile Leu Ser Asn Asp Ala Leu Arg
       290                 295                 300
   gcg att tgg gaa caa gag ctg act gat atg cgc cag cgt att cag cgt    960
   Ala Ile Trp Glu Gln Glu Leu Thr Asp Met Arg Gln Arg Ile Gln Arg
   305                 310                 315                 320
   atg cgt cag ttg ttc gtc aat acg ctg cag gaa aaa ggc gca aac cgc    1008
   Met Arg Gln Leu Phe Val Asn Thr Leu Gln Glu Lys Gly Ala Asn Arg
                   325                 330                 335
   gac ttc agc ttt atc atc aaa cag aac ggc atg ttc tcc ttc agt ggc    1056
   Asp Phe Ser Phe Ile Ile Lys Gln Asn Gly Met Phe Ser Phe Ser Gly
               340                 345                 350
   ctg aca aaa gaa caa gtg ctg cgt ctg cgc gaa gag ttt ggc gta tat    1104
   Leu Thr Lys Glu Gln Val Leu Arg Leu Arg Glu Glu Phe Gly Val Tyr
           355                 360                 365
   gcg gtt gct tct ggt cgc gta aat gtg gcc ggg atg aca cca gat aac    1152
   Ala Val Ala Ser Gly Arg Val Asn Val Ala Gly Met Thr Pro Asp Asn
       370                 375                 380
   atg gct ccg ctg tgc gaa gcg att gtg gca gtg ctg taa                1191
   Met Ala Pro Leu Cys Glu Ala Ile Val Ala Val Leu
   385                 390                 395
<210> 2
   <211> 396
   <212> PRT
   <213> Escherichia coli
<400> 2
   Met Phe Glu Asn Ile Thr Ala Ala Pro Ala Asp Pro Ile Leu Gly Leu
    1               5                  10                  15
   Ala Asp Leu Phe Arg Ala Asp Glu Arg Pro Gly Lys Ile Asn Leu Gly
               20                  25                  30
   Ile Gly Val Tyr Lys Asp Glu Thr Gly Lys Thr Pro Val Leu Thr Ser
           35                  40                  45
   Val Lys Lys Ala Glu Gln Tyr Leu Leu Glu Asn Glu Thr Thr Lys Asn
       50                  55                  60
   Tyr Leu Gly Ile Asp Gly Ile Pro Glu Phe Gly Arg Cys Thr Gln Glu
   65                  70                  75                  80
   Leu Leu Phe Gly Lys Gly Ser Ala Leu Ile Asn Asp Lys Arg Ala Arg
                   85                  90                  95
   Thr Ala Gln Thr Pro Gly Gly Thr Gly Ala Leu Arg Val Ala Ala Asp
               100                 105                 110
   Phe Leu Ala Lys Asn Thr Ser Val Lys Arg Val Trp Val Ser Asn Pro
           115                 120                 125
   Ser Trp Pro Asn His Lys Ser Val Phe Asn Ser Ala Gly Leu Glu Val
       130                 135                 140
   Arg Glu Tyr Ala Tyr Tyr Asp Ala Glu Asn His Thr Leu Asp Phe Asp
   145 150 155 160
   Ala Leu Ile Asn Ser Leu Asn Glu Ala Gln Ala Gly Asp Val Val Leu
   165 170 175
   Phe His Gly Cys Cys His Asn Pro Thr Gly Ile Asp Pro Thr Leu Glu
   180 185 190
   Gln Trp Gln Thr Leu Ala Gln Leu Ser Val Glu Lys Gly Trp Leu Pro
   195 200 205
   Leu Phe Asp Phe Ala Tyr Gln Gly Phe Ala Arg Gly Leu Glu Glu Asp
   210 215 220
   Ala Glu Gly Leu Arg Ala Phe Ala Ala Met His Lys Glu Leu Ile Val
   225 230 235 240
   Ala Ser Ser Tyr Ser Lys Asn Phe Gly Leu Tyr Asn Glu Arg Val Gly
   245 250 255
   Ala Cys Thr Leu Val Ala Ala Asp Ser Glu Thr Val Asp Arg Ala Phe
   260 265 270
   Ser Gln Met Lys Ala Ala Ile Arg Ala Asn Tyr Ser Asn Pro Pro Ala
   275 280 285
   His Gly Ala Ser Val Val Ala Thr Ile Leu Ser Asn Asp Ala Leu Arg
   290 295 300
   Ala Ile Trp Glu Gln Glu Leu Thr Asp Met Arg Gln Arg Ile Gln Arg
   305 310 315 320
   Met Arg Gln Leu Phe Val Asn Thr Leu Gln Glu Lys Gly Ala Asn Arg
   325 330 335
   Asp Phe Ser Phe Ile Ile Lys Gln Asn Gly Met Phe Ser Phe Ser Gly
   340 345 350
   Leu Thr Lys Glu Gln Val Leu Arg Leu Arg Glu Glu Phe Gly Val Tyr
   355 360 365
   Ala Val Ala Ser Gly Arg Val Asn Val Ala Gly Met Thr Pro Asp Asn
   370 375 380
   Met Ala Pro Leu Cys Glu Ala Ile Val Ala Val Leu
   385 390 395
<210> 3
   <211> 36
   <212> DNA
   <213> Artificial Sequence
<220>
   <223> Description of Artificial Sequence:primer
<400> 3
   gctacttacg aattccgttt gtcatcagtc tcagcc 36
<210> 4
   <211> 36
   <212> DNA
   <213> Artificial Sequence
<220>
   <223> Description of Artificial Sequence:primer
<400> 4
   cctagatcac agctgatgtt tgagaacatt accgcc 36
<210> 5
   <211> 36
   <212> DNA
   <213> Artificial Sequence
<400> 5
   ttgactattt tacctctggc ggtgataatg gtccca 36
<210> 6
   <211> 40
   <212> DNA
   <213> Artificial Sequence
<400> 6
   agcttgggac cattatcacc gccagaggta aaatagtcaa 40